# EUROPEAN PATENT APPLICATION

(11) **EP 3 219 793 A1**
(43) Date of publication of application: **20.09.2017**
(21) Application number: 17168133.1
(22) Date of filing: 27.12.2012
(51) Int. Cl.: C12N 5/0775, A61K 35/28

(54) **PREPARATION OF CELL TRANSPLANT**

(30) Priority: 05.01.2012 US 201261583367 P
(62) Divisional of application: 12199521.1
(71) Applicant: Taipei Veterans General Hospital, Taipei City, Taiwan 11217 (TW)
(72) Inventor: Hung, Shih-Chieh, Taipei City 112 (TW); Yew, Tu-Lai, Taipei City 112 (TW); Huang, Wei-Hua, Taipei City 112 (TW)
(74) Representative: Becker Kurig Straus

(57) **Abstract**

The preset invention relates to a novel cell graft suitable for transplantation or cell therapy and the use thereof for treating diseases. In particular, the graft provided herein overcomes concomitant rejection when transplanted onto a patient, which is especially beneficial for the condition where allogeneic cells are used. Also provided is a process for preparing such grafts by culturing at least one cell type under hypoxic or anoxic conditions.

## Description

This application claim benefit under 35 U.S.C 119(e) of U.S. Provisional Application No. 61/583,367, filed January 5, 2012, the entire content of which are incorporated by reference herein.

### FIELD OF THE INVENTION

The present invention relates to a method for preparing allogeneic cell-preparing-tissue-engineering grafts and products prepared by the method.

### BACKGROUND OF THE INVENTION

Mesenchymal stem cells (MSCs), also termed multipotent stromal cells, which were known to be capable of self-renewal and differentiating into various mesenchymal and non-mesenchymal tissues, have already emerged as a promising tool for clinical applications in, for example, cell-based therapy for osteogenesis imperfecta (Horwitz et al., Nat Med 5, 309-313, 1999), tissue engineering in cartilage and bone (Caplan, Tissue Eng 11, 1198-1211, 2005), and cardiac therapeutics by preventing deleterious remodeling and improving recovery (Pittenger and Martin, Circ Res 95, 9-20, 2004).

Use of an "off the-shelf" product from the MSCs of a single healthy donor cell has proven safe in two phase III studies in patients with graft-versus-host disease and Crohn's disease (http://www.clinicaltrails.gov/). Moreover, improvement in ejection fractions (EFs) was also reported in acute myocardial infarction patients treated with allogeneic MSCs (Hare JM, et al., J Am Coll Cardiol. 54(24):2277-2286, 2009). Thus, allogeneic MSCs from healthy donors may serve as a "universal donor cell" in treating patients with cardiovascular diseases. The immunosuppressive features of human, baboon and murine MSCs were also reported in vitro, and in vivo, where allogeneic MSCs were applied for prolonging skin allograft survival in immunocompetent baboons (Bartholomew et al., Exp Hematol. 30(1):42-48, 2002), and attenuating graft-versus-host disease in humans (Le Blanc et al., Lahcet. 363(9419):1439-1441, 2004). However, a lot of studies have demonstrated that MSCs are not intrinsically immunoprivileged and allogeneic MSCs were rejected in immunocompetent MHC-mismatched recipients (Nauta et al., Blood 108(6): 2114-2120,2006; Spaggiari et al., Blood. 107(4):1484-1490, 2006; Eliopoulos et al., Blood 106(13):4057-4065, 2005). Thus, it remains controversy in using MSCs for allogeneic transplantation.

MSCs cultured under low oxygen conditions, here referred to as hypoxic MSCs, secrete more angiogenic cytokines and growth factors than MSCs cultured under normal oxygen conditions (at about 20-21% O₂), here referred to as normoxic MSCs (Hung et al., Stem Cells 25(9):2363-2370, 2007) and the conditioned medium derived from hypoxic MSCs have been applied for stimulating wound healing (Yew et al., Cell Transplant., 2011) and fracture healing (Wang et al., J Tissue Eng Regen Med., 2011).

In addition, Savant-Bhonsale and Smita disclosed a method and composition for enhancing the growth of neural stem cells (NSCs) under low oxygen conditions ranging from about 2.5% through about 5% oxygen (O₂), which is lower than the environmental oxygen conditions traditionally employed in cell culture techniques (US20070264712 A1). It was reported by Hung *et al.* that MSCs cultured under the low-density and low oxygen conditions with 0.05% to 15% O₂ (preferably ranging from about 1 % to about 7% oxygen), here referred to as hypoxic and low-density MSCs (10 to 4000 MSCs/cm²), decrease replicative senescence and increased proliferation rate and differentiation potential, as compared to normoxic MSCs (US20110129918A1). Moreover, these hypoxic MSCs promote defect repair when transplanted into calvarial defect of immunodeficient mice. Hypoxic and low-density MSCs also enhanced the migration and engraftment to distant sites following transplantation (Hung et al., PLoS One. 2(5):e416, 2007). However, there is no mention regarding the solution to the problem of rejection.

### SUMMARY OF THE INVENTION

The present invention relates to a new approach for reducing rejection of allogeneic cells by culturing the cell under hypoxic or anoxic conditions.

In one aspect, the invention provides a cell graft suitable for transplanting onto a subject, which comprises non-naturally occurring cells with reduced expression of NK ligands.

In another aspect, further provided is a process for preparing cell grafts which induce reduced or no graft rejection when transplanted onto a subject, comprising culturing at least one cell type isolated from a donor under low oxygen conditions ranging from 0% to about 7 % oxygen.

In one preferable embodiment of the invention, the low oxygen conditions ranges from 0% to about 2.5% oxygen. In one example of the invention, the low oxygen condition is about 1% oxygen.

In further aspect, the invention provides a cell graft prepared by the aforementioned process. According to the invention, the cell graft as obtained preserve early stem cell properties, maintain normal karyotyping, and will not form tumor after transplantation.

### BRIEF DESCRIPTION OF THE DRAWINGS

The foregoing summary, as well as the following detailed description of the invention, will be better understood when read in conjunction with the appended drawings. For the purpose of illustrating the invention, they are shown in the drawings embodiments which are presently preferred. It should be understood, however, that the invention is not limited to the embodiments shown.

In the drawings:

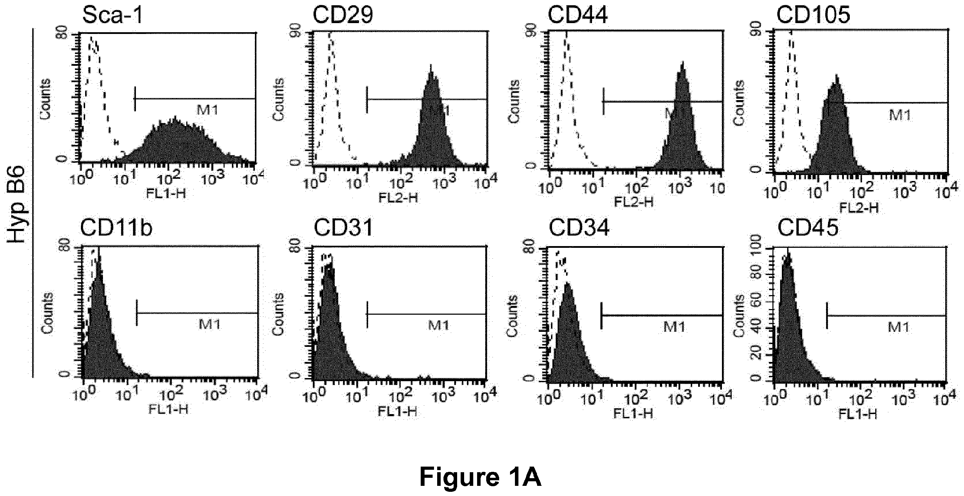

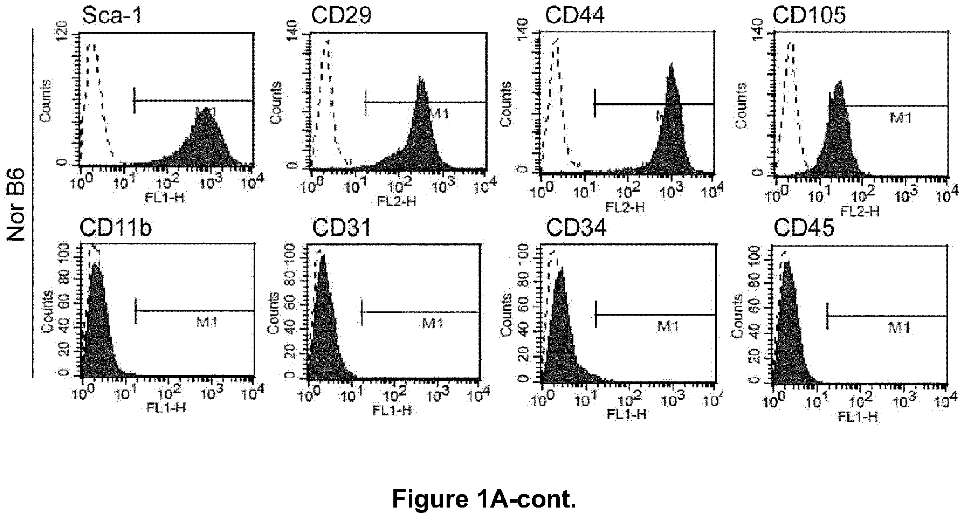

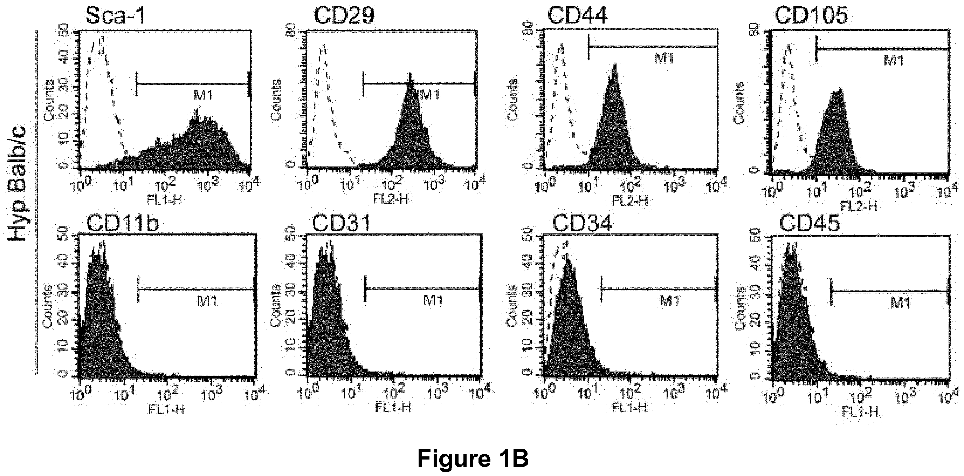

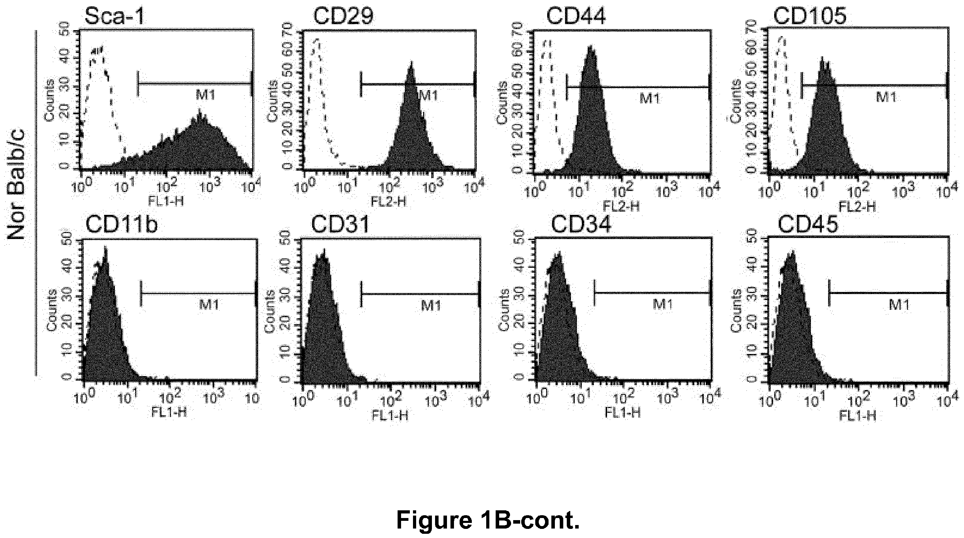

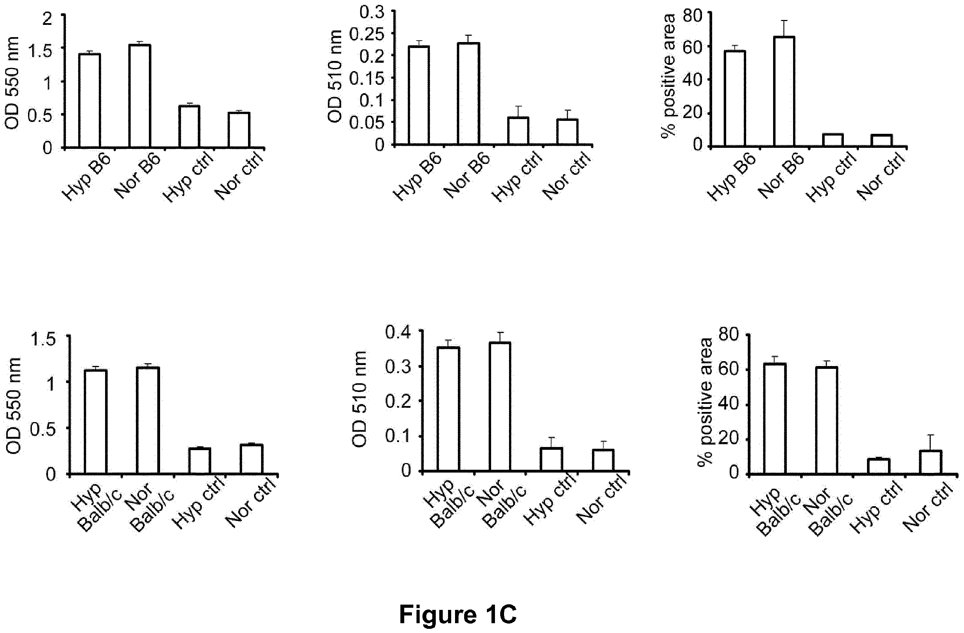

Figure 1 provides the characterization of normoxic (Nor) and hypoxic (Hyp) mesenchymal stem cells (MSCs), wherein the MSCs from B6 (A) and Balb/c (B) were characterized for the expression of surface markers; and (C) shows the differentiation potency of the MSCs from both strains induced for osteogenic (21 days), adipogenic (21 Days) and chondrogenic lineages (21 Days), which were analyzed with Alizarin Red S, Oil-red O and Alcian Blue staining, respectively (Bar= 50 µm). Quantitative analysis of Alizarin Red S, Oil-red O and Alcian Blue staining was performed by optical density measurement at 550 nm, 510 nm and positive area measurement, respectively.

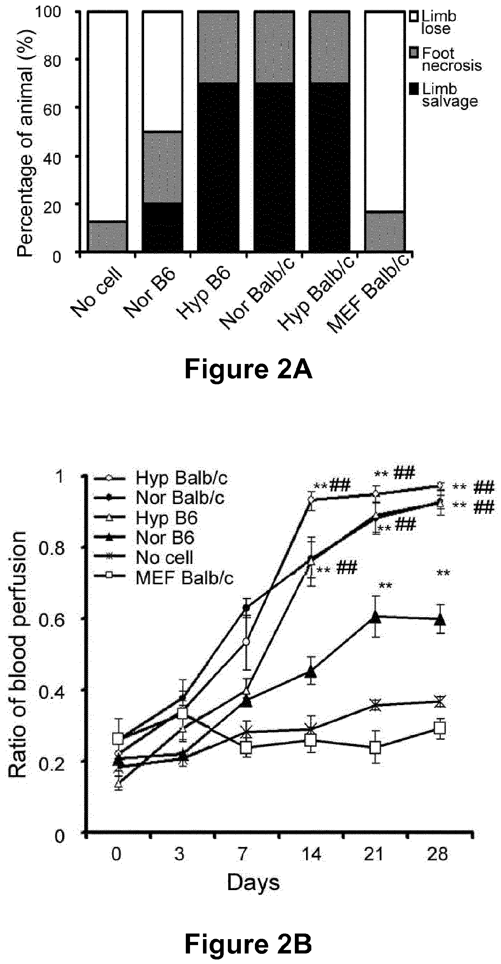

Figure 2 provides the effect of hypoxic MSCs in improving limb ischemia both in allogeneic and syngeneic transplantation, wherein Balb/c mice with left femoral artery truncated were i.m. injected without (No cell) or with 1×10⁶ normoxic B6 MSCs (Nor B6; allogeneic), hypoxic B6 MSCs (Hyp B6; allogeneic), normoxic Balb/c MSCs (Nor Balb/c; syngeneic), hypoxic Balb/c MSCs (Hyp Balb/c; syngeneic) or Balb/c mouse embryonic fibroblasts (MEF Balb/c) in the Gracilis muscle. Figure 2A summarizes the limb status of the animals at 28 days; and Figure 2B shows the blood perfusion indices for indicated time period (**p < 0.01 versus No cell, ##p < 0.01 versus Nor B6).

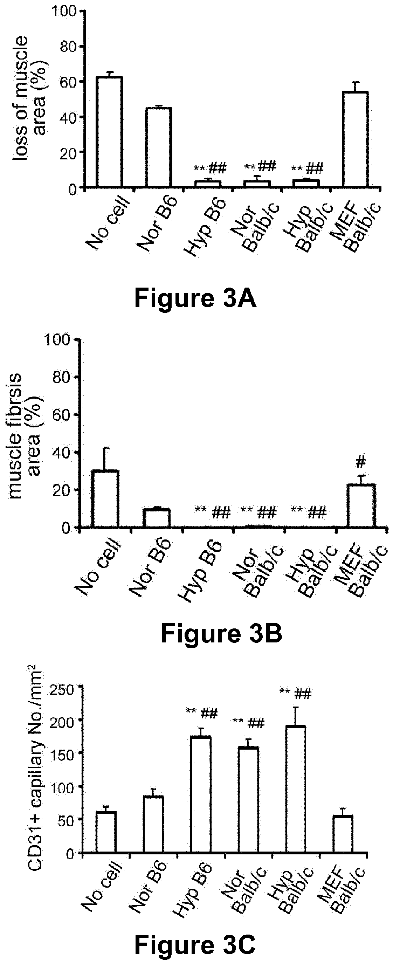

Figure 3 provides the effect of hypoxic MSCs in decreasing the muscle degeneration and fibrosis and increasing the blood perfusion of ischemic limb, wherein Figure 3A shows the quantitative measurement of the loss of muscle area by HE staining; Figure 3B shows the quantitative measurement of the muscle fibrosis area by Masson's trichrome staining; and Figure 3C shows the quantitative measurement of the CD31+ capillary density of 28-day ischemic limb specimens by immunohistochemistry using an antibody against CD31 (*p < 0.05 and **p < 0.01 versus No cell, #p < 0.05 and ##p < 0.01 versus Nor B6.).

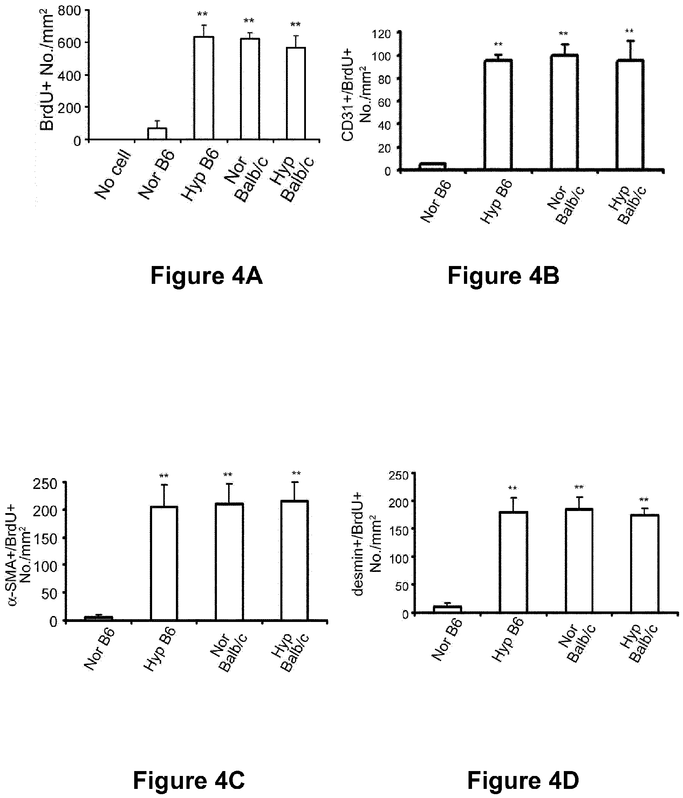

Figure 4 shows that the hypoxic MSCs survived 28 days after transplantation, wherein Balb/c mice with left femoral artery truncated were i.m. injected without (No cell) or with 1×10⁶ BrdU-incorporated B6 normoxic MSCs (Nor B6; allogeneic), B6 hypoxic MSCs (Hyp B6; allogeneic), Balb/c normoxic MSCs (Nor Balb/c), or Balb/c hypoxic MSCs (Hyp Balb/c; syngeneic) in the Gracilis muscle; the specimens of ischemic limbs were harvested 28 days later, wherein Figure 4A shows the immunofluorescence using antibody against BrdU; and Figure 4B-D show the double immunofluorescence using antibodies against BrdU and (Figure 4B) CD31, (Figure 4C) α-smooth muscle actin, and desmin (Figure 4D), respectively.

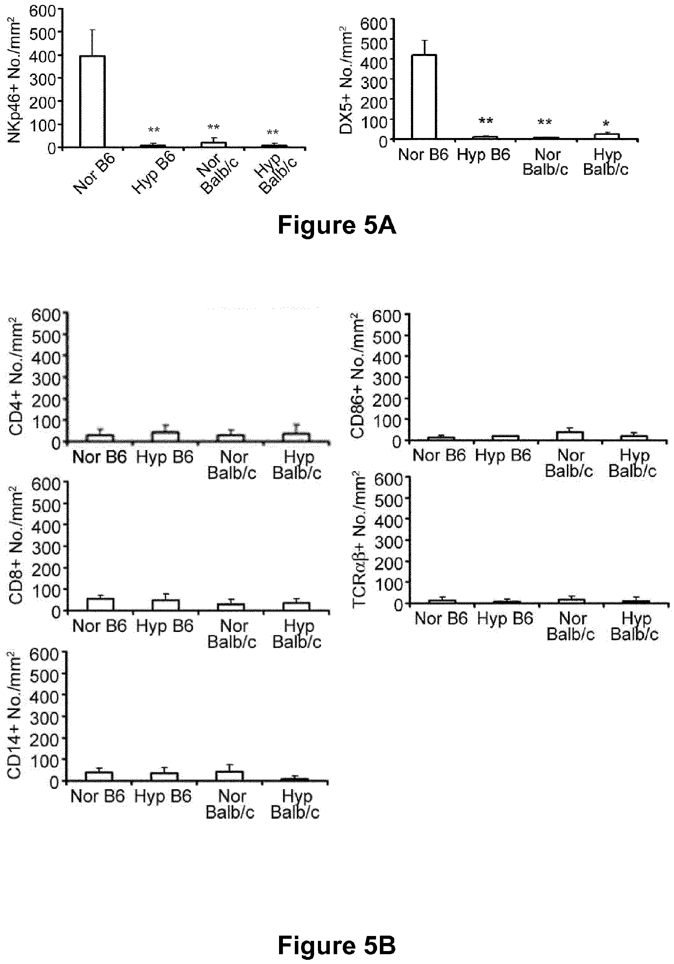

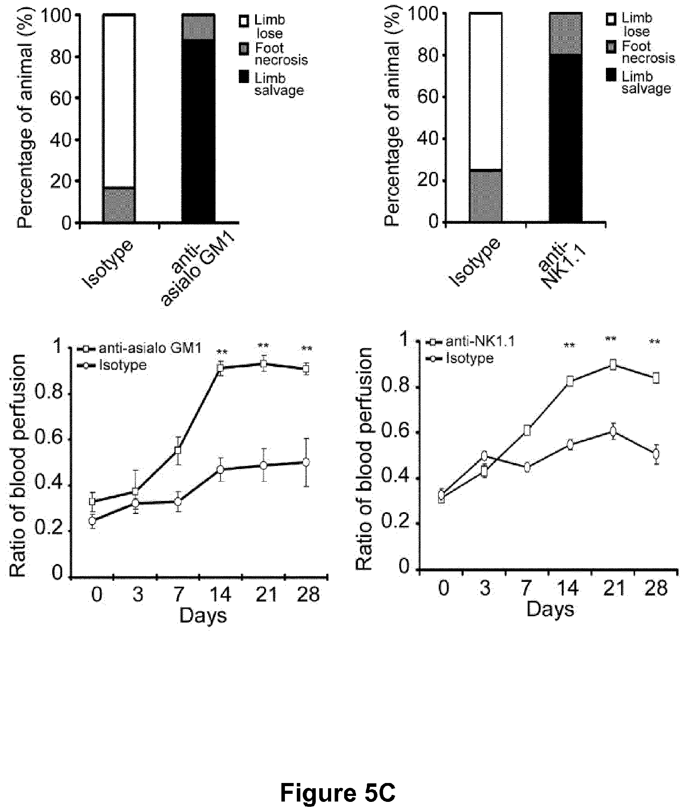

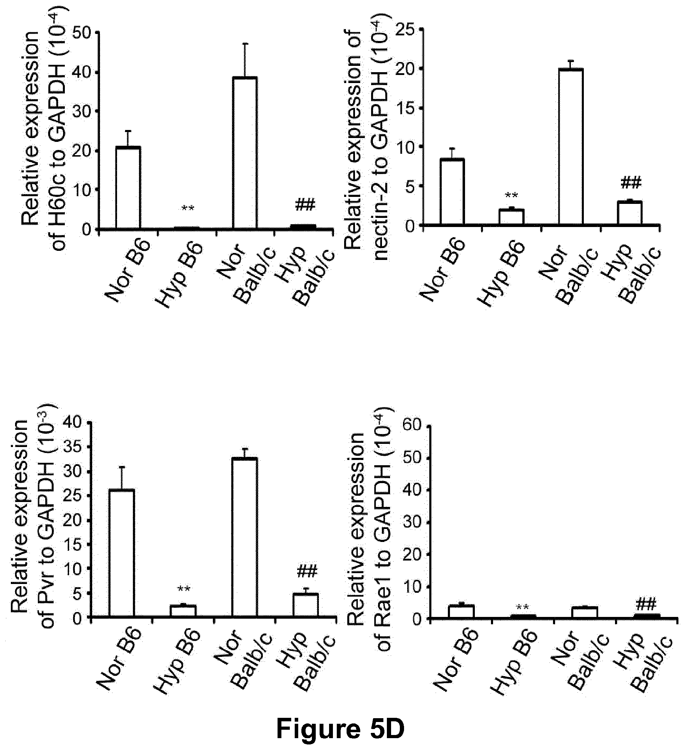

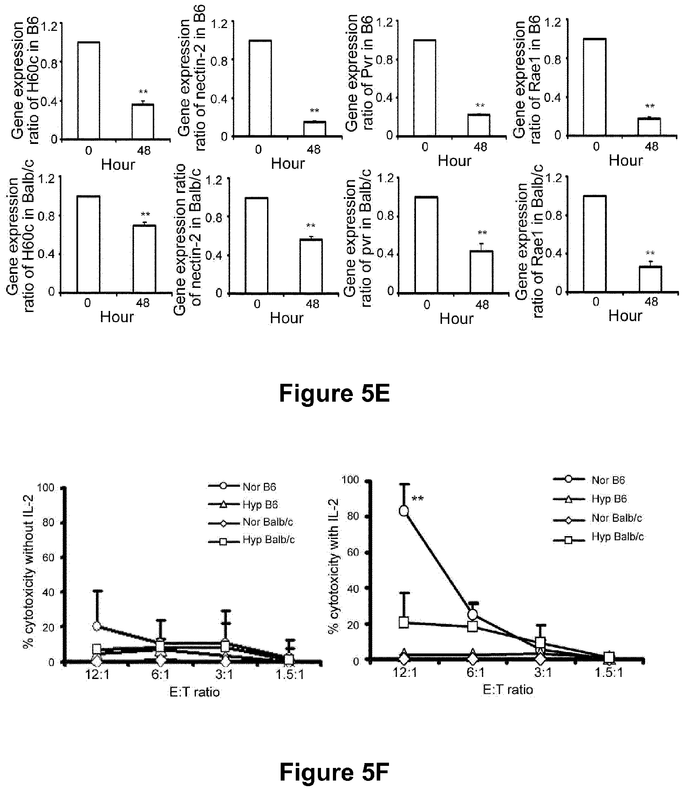

Figure 5 shows that hypoxic MSCs decrease expression of ligands for NK activation and NK-mediated lysis, wherein Figures 5A shows the statistical results of immunofluorescence with antibody against NKp46 of 28-day (left panel) and DX5 of 7-day (right panel) ischemic limb specimens of mice injected with 10⁶ B6 normoxic MSCs (Nor B6), B6 hypoxic (Hyp B6), Balb/c normoxic MSCs (Nor Balb/c), or Balb/c hypoxic MSCs (Hyp Balb/c) (**p < 0.01 versus Nor B6); Figure 5B shows the statistical results of immunofluorescence with antibody against CD4, CD8, CD14, CD86, or TCRαβ of 7-day ischemic limb specimens of mice injected with 10⁶ B6 normoxic MSCs (Nor B6), B6 hypoxic (Hyp B6), Balb/c normoxic MSCs (Nor Balb/c), or Balb/c hypoxic MSCs (Hyp Balb/c) (**p < 0.01 versus Nor B6); Figure 5C shows the status of the limb (upper panel) and ratio of blood perfusion for indicated time period (lower panel), in which Balb/c mice with truncated left femoral artery at Day 28 after being co-injected with 10⁶ B6 normoxic MSCs and indicated antibodies are shown in the left and B6 mice with truncated left femoral artery at Day 28 after being co-injected with 10⁶ Balb/c normoxic MSCs and indicated antibodies are shown in the right; Figure 5D shows the quantitative RT-PCR analyses of the mRNA levels of H60c to GAPDH (10⁻⁴), nectin-2 to GAPDH (10⁻⁴), Pvr to GAPDH (10⁻³), and Rae1 to GAPDH (10⁻⁴) in indicated cells (**p < 0.01 versus Nor B6, ^{##}p < 0.01 versus Nor Balb/c); and Figure 5E shows the quantitative RT-PCR analyses of the mRNA levels of H60c, nectin-2, Pvr, and Rae1 in B6 and Balb/c MSCs, which were seeded at 10⁴/cm² and exposed to hypoxic culture (1% O₂) for 48 h (**p < 0.01 versus 0 h); and Figure 5F shows the cytotoxic activities of NK cells to MSCs at different E:T ratios after co-culturing for 4 h, wherein NK cells were first expanded for 5 days without (left panel) or with (right panel) IL-2 before co-culture of NK cells and MSCs.

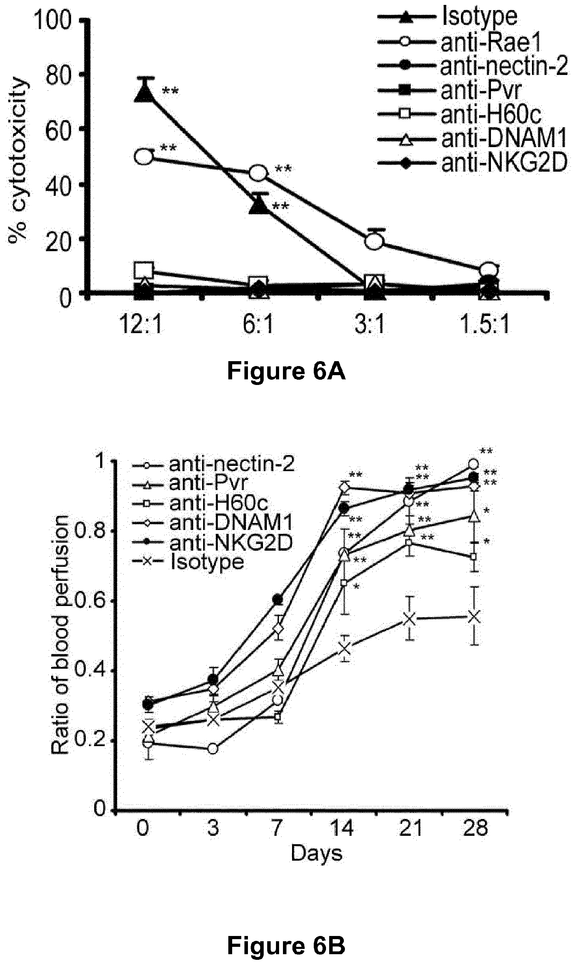

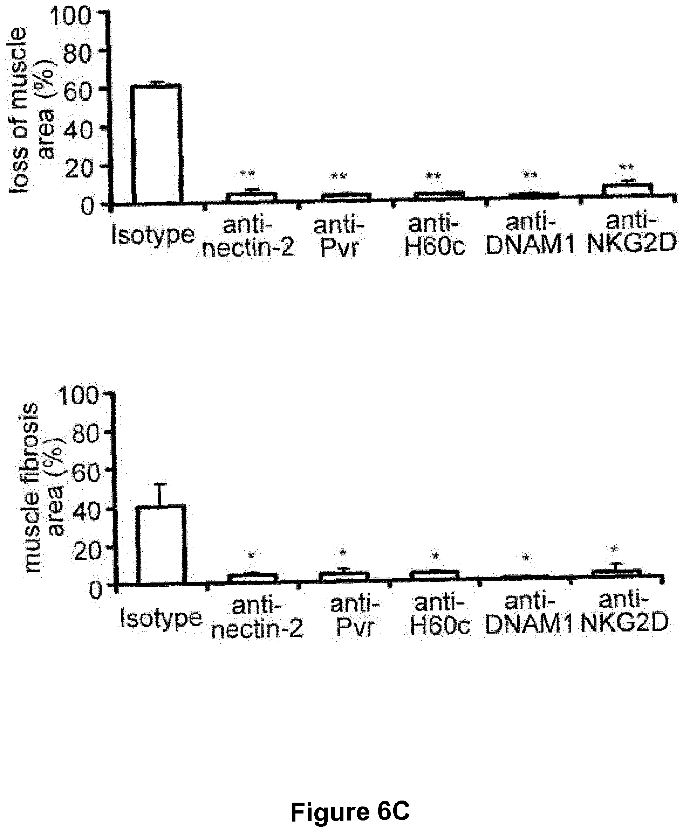

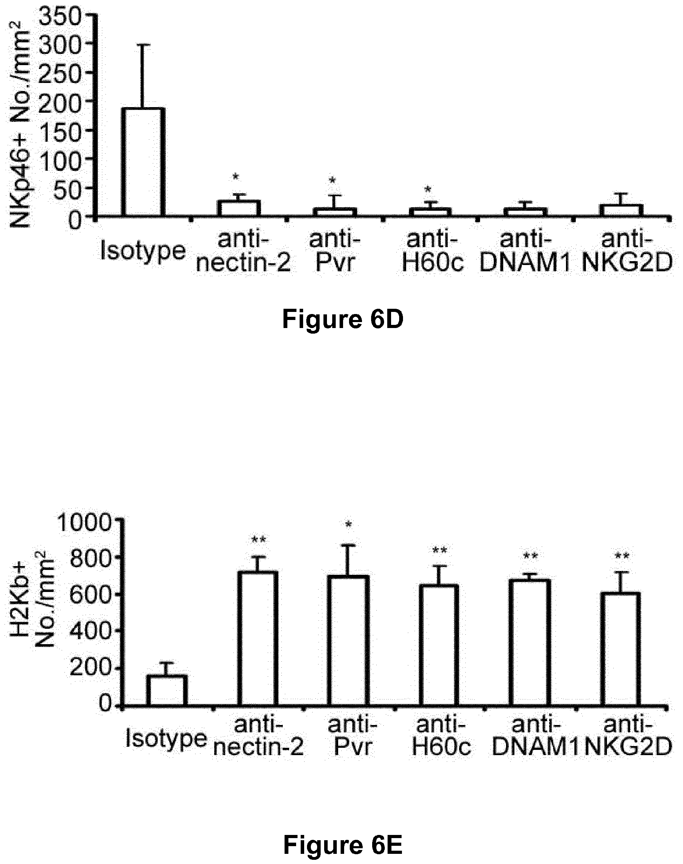

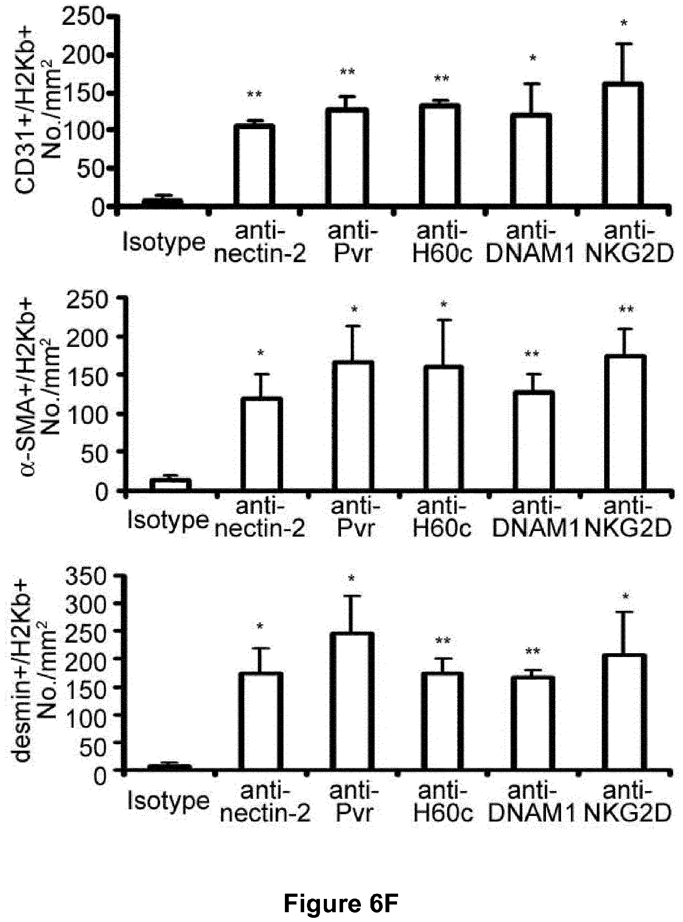

Figure 6 shows that the neutralization of normoxic B6 MSCs with antibody against NK ligand antibodies inhibited NK-mediated lysis and improved their effect in enhancing blood perfusion and restoring muscle structure in ischemic limbs, wherein Figure 6A shows the cytotoxic activities of NK cells, which were first expanded for 5 days with IL-2, to MSCs at different E:T ratios after co-culturing for 4 h; Figures 6B and 6C show that Balb/c mice with left femoral artery truncated were i.m. injected with 10⁶ B6 normoxic MSCs pre-incubated with isotype antibodies or antibodies against each indicated ligand for 30 min, wherein Figure 6B provides the blood perfusion indices for indicated time period and Figure 6C provides the quantitative measurement of the loss of muscle area and the muscle fibrosis area from H.E. and Masson's trichrome stain; Figures 6D-6F provide the statistical results of immunofluorescence with antibody against (D) NKp46, (E) H2Kb, and of double-immunostaining with antibody against (F) H2Kb and CD31, H2Kb and α-SMA or H2Kb and desmin (densities of immunostained cells are shown, * p < 0.05, **p < 0.01 versus isotype IgG).

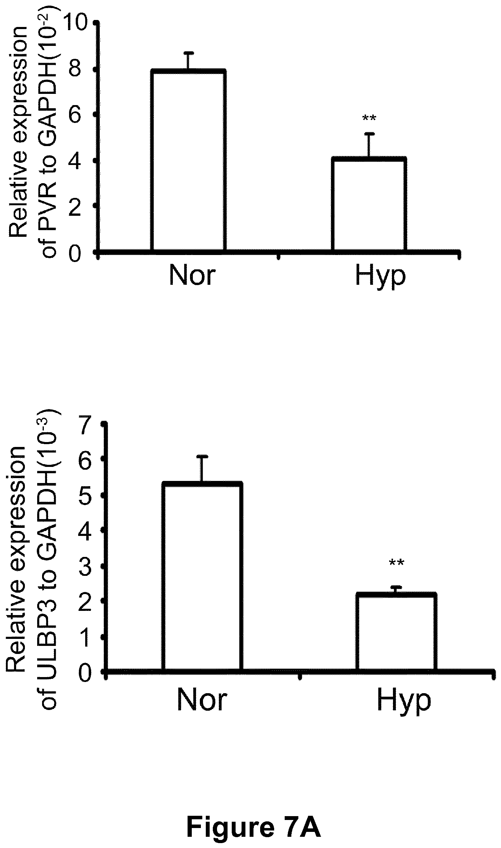

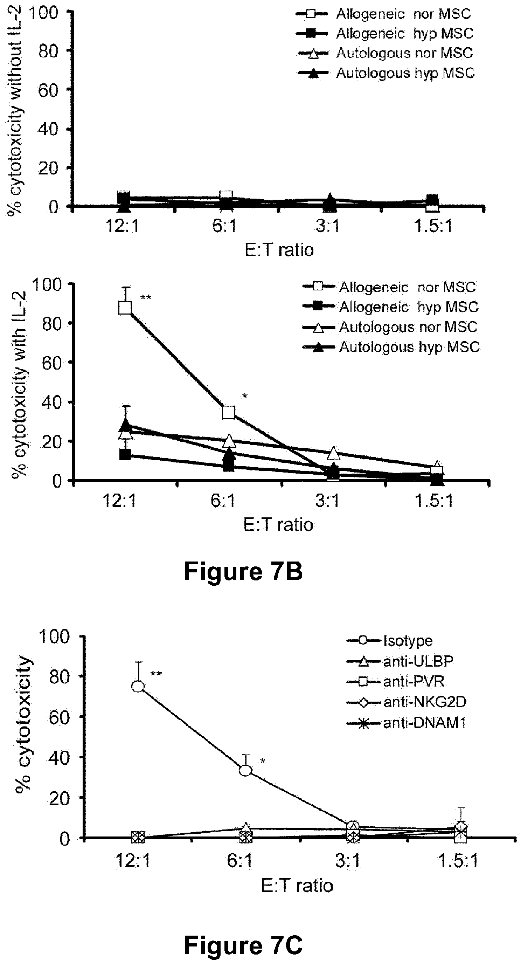

Figure 7 shows that the human MSCs cultured under hypoxic conditions decreased in the expression of human NK ligands. In this study, human MSCs were seeded at a density of 5 ×10³ MSCs/cm² and half of the cells were continuously cultured under normoxic conditions or exposed to hypoxic conditions (1% O₂) for 48 h. Quantitative RT-PCR for the mRNA levels of two NK ligands, PVR and ULBP3, were performed (**p < 0.01 versus Isotype Nor).

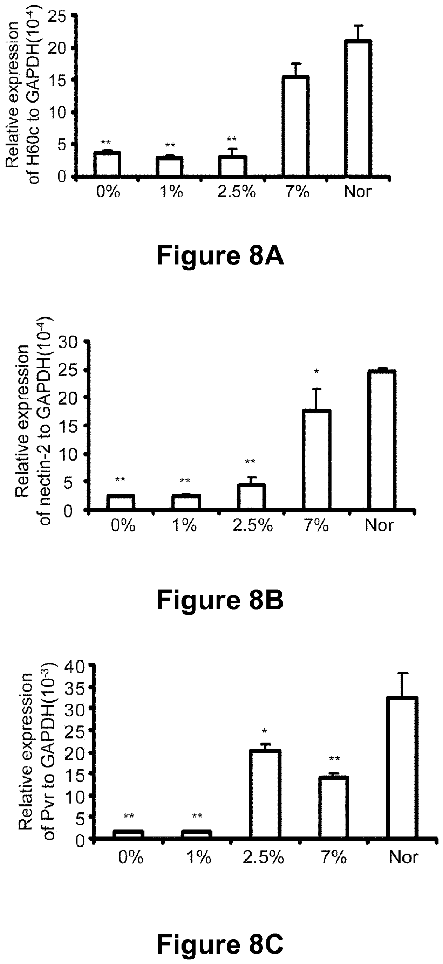

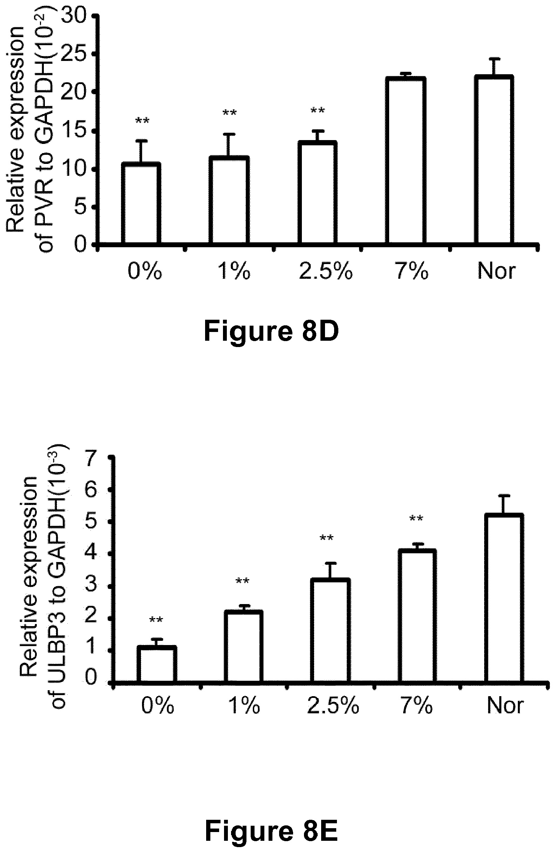

Figure 8 shows that anoxic and hypoxic MSCs (0%, 1%, 2.5%, 7% oxygen conditions) decreased NK accumulation in allogeneic recipients of mice as compared to normoxic (Nor) MSCs, wherein Figures 8A shows the relative expression of H60c to GAPDH (10⁻⁴); Figure 8B shows the relative expression of nectin-2 to GAPDH(10⁻⁴); Figure 8C shows the relative expression of Pvr to GAPDH (10⁻³); Figure 8D shows the relative expression of PVR to GAPDH (10⁻²); and Figure 8E shows the relative expression of ULBP3 to GAPDH (10⁻³) (* p < 0.05, **p < 0.01 versus Nor B6).

### DESCRIPTION OF THE INVENTION

Unless defined otherwise, all technical and scientific terms used herein have the same meaning as commonly understood by a person skilled in the art to which this invention belongs.

As used herein, the singular forms "a", "an", and "the" include plural referents unless the context clearly dictates otherwise. Thus, for example, reference to "a sample" includes a plurality of such samples and equivalents thereof known to those skilled in the art.

The term "mesenchymal stem cells" or "MSCs" as used herein, which is also termed "multipotent stromal cells" or "multipotent stem cells", refers to the stem cells that can differentiate into variety of cell types, including the cells of mesenchymal and non-mesenchymal tissues, such as osteoblasts (bone cells), chondrocytes (cartilage cells) and adipocytes (fat cells). The MSCs can be derived from bone marrow, or other non-marrow tissues, such as umbilical cord blood, adipose tissue, adult muscle, umbilical cord or amniotic membrane, dental pulp of deciduous baby teeth and etc. In the invention, the MSCs are of mammalian origin, preferably those isolated from humans.

The term "hypoxic" as used herein refer to a low oxygen condition that is lower than the ambient oxygen conditions or the oxygen conditions traditionally employed in cell culture techniques, about 20%-21% oxygen. In one embodiment of the invention, the "hypoxic condition" is less than 7% oxygen, preferably ranging from 0% to about 7% oxygen, more preferably ranging from 0% to about 2.5% oxygen, and most preferably about 1% oxygen. In addition, the term "anoxic" as used herein refers to an oxygen condition completely depleted of oxygen (0% O₂), which is an extreme condition of hypoxic conditions or "low oxygen conditions".

The term "normoxic" as used herein refers to an ambient oxygen condition around 20%-21% oxygen, which is traditionally employed in cell culture techniques.

The term "subject" as used herein is a human or non-human mammal. Non-human mammals include, but are not limited to, primates, ungulates, canines and felines.

The invention is based on the unexpected finding that MSCs expanded in low oxygen conditions expressed relatively low expression of NK ligands as compared to those expanded in normal condition with an oxygen level ranging from, for example, 20% to 21 %. More surprisingly, these cells successfully rescued limb ischemia in a mouse model without inducing transplantation rejection, which provides a promising solution to the long-standing problem regarding the use of allogeneic cells for cell or transplantation therapy.

Accordingly, the invention provides a cell graft suitable for transplanting onto a subject, which comprises non-naturally occurring cells with reduced expression of NK ligands. The cell graft as provided herein produces reduced or no graft rejection when transplanted onto a subject.

"Natural killer cells" or "NK cells" are a type of cytotoxic lymphocyte critical to the innate immune system. The role NK cells play is analogous to that of cytotoxic T cells in the vertebrate adaptive immune response. NK cells provide rapid responses to virally infected cells and respond to tumor formation. Typically immune cells detect MHC presented on infected cell surfaces, triggering cytokine release causing lysis or apoptosis. NK cells are different from typical immune cells because they have the ability to recognize stressed cells in the absence of antibodies and MHC, allowing for a much faster immune reaction.

The term "NK ligand" as used herein refers to any molecule or substance that interacts with activating NK receptors and, once engaged, induces both cytotoxicity and lymphokine release. Examples of activating NK receptors in accordance with the invention are DNAM1 and NKG2D. The NK ligands that interact with these receptors and may be used in the invention include, but are not limited to, H60c, nectin-2, PVR, ULBP3, an analogue thereof and combinations thereof.

H60c is a MHC class I-like molecule with two extracellular domains and serves as a ligand for the NKG2D receptor.

Nectin-2 or PVRL2 (poliovirus receptor-related 2), also known as CD112 (Cluster of Differentiation 112), is a single-pass type I membrane glycoprotein with two Ig-like C2-type domains and an Ig-like V-type domain. This protein is one of the plasma membrane components of adherens junctions. It also serves as an entry for certain mutant strains of herpes simplex virus and pseudorabies virus, and it is involved in cell to cell spreading of these viruses.

PVR (or Pvr in mouse), also known as CD155 (Cluster of Differentiation 115) is a human type I transmembrane glycoprotein in the immunoglobulin superfamily. Commonly known as Poliovirus Receptor (PVR) due to its involvement in the cellular poliovirus infection in primates, CD155's normal cellular function is in the establishment of intercellular adherens junctions between epithelial cells.

ULBP3, also known as NKG2D ligand 3, is a stress induced ligand in human for the NK cell activating receptor NKG2D.

According to the present invention, the term "reduced expression" as it applies to the expression of NK ligands will include those situations where the level of said marker or markers expressed by a non-naturally occurring cell (such as one cultured in vitro under a particular condition) is lower than that expressed by a naturally occurring cell or a cell cultured by traditional culture techniques. For example, "reduced expression" may refer to a level of 80%, preferably refer to a level of 60%, more preferably refer to a level less than 50%, or even more preferably to a level of 20% as compared to the level expressed by a cell residing in a human body or a cell cultured under a normal condition. It is to be understood that the expression of said marker or markers can be suppressed by any one of the techniques known to a person of ordinary skill in the art. For example, the expression may be suppressed or eliminated by gene disruption or deletion. In one embodiment of the invention, a cell is expanded under a low oxygen (hypoxic or anoxic) condition (containing 0% to 7% oxygen, for example) and recovered after achieving semi- to full-confluence such that the expression of NK ligands is reduced as compared to the cell expanded under a normal oxygen (normoxic) condition (containing 20% to 21% oxygen, for example). In one preferable embodiment of the invention, the low oxygen condition is 1% O₂.

It was also unexpectedly found that any density of MSCs, either a high density or a low density, may be used in accordance with the invention, to the contrary to the low density of 10 to 4000 MSCs/cm² being a patentable characteristic of the invention as disclosed in US20110129918A1. In one example of the invention, the density of 50 cells/cm² was used. In the other example, 5×10³ to 10⁴ cells/cm² was used.

It was demonstrated in the example of the present invention that allogeneic hypoxic MSCs improved the therapeutic effects in attenuating limb ischemia as compared to allogeneic normoxic MSCs, and the effects were similar with autologous hypoxic MSCs. The allogeneic hypoxic MSCs according to the invention increased the ability to engraft in immunocompetent recipient mice via suppression of NK ligands, such as ligands associated with DNAM1 and NKG2D on NK cells and reduction in inducing NK recruitment and cytotoxicity. These cells gave rise to CD31+ endothelial cells and αSMA⁺ and desmin⁺ muscle cells, thereby enhancing angiogenesis and inhibiting muscle fibrosis, which suggests that the hypoxic MSCs according to the invention are intrinsically immunoprivileged and can serve as a "universal donor cell" for treating cardiovascular diseases.

It is known that MSCs, similar with hematopoietic stem cells (HSCs) or other bone marrow grafts, are also rejected by NK mediated lysis in allogeneic recipients; however, the hypoxic MSCs according to the invention (which was obtained by culturing MSCs under hypoxic conditions) decreased the expression ofNK ligands and inhibited recruitment and cytotoxicity ofNK cells, thereby enhancing the ability to survive and engraft into tissues of allogeneic recipients.

In the invention, the allogeneic hypoxic MSCs used for cell therapy has been tested in an immunocompetent animal model as a preclinical study, showing its therapeutic potential and immune benefit, such as in the treatment of severe ischemic diseases. The results shows that the allogeneic hypoxic MSCs according to the invention preserves early stem cell properties, maintains normal karyotyping, and will not form tumor after transplantation. Therefore, the MSCs cultured under hypoxic conditions according to the invention can be used for allogeneic transplantation.

In yet aspect, the invention also provides a process for preparing cell grafts which induce reduced or no graft rejection when transplanted onto a subject, comprising culturing at least one cell type isolated from a donor under low oxygen conditions ranging from 0% to 15% oxygen, preferably ranging from 0% to about 7 % oxygen, more preferably ranging from 0% to about 2.5 % oxygen. In one example of the invention, the low oxygen condition is about 1% oxygen.

It is to be understood that the at least one cell type used in the cell graft in accordance with the invention may be selected based on a target transplantation site. Examples of suitable cells for use in the invention include various stem cells which are capable of self-renewal and differentiation. In one preferable embodiment of the invention, mesenchymal stem cells (MSCs) are used. It is indicated that the hypoxic MSCs according to the invention could be an alternative cell source for use in therapeutic angiogenesis to treat ischemic diseases, at least for the following reasons:
(1) These cells could provide functional endothelial and muscular cells in unlimited amounts that would be available for clinical application in comparison with the limited number of endothelial progenitor cells in adult bone marrow or peripheral blood. Furthermore, the endothelial cells or progenitor cells usually have a limited proliferative capacity, and thus, expansion of sufficient cell number for transplantation remains a major task; in contrast, the hypoxic MSCs according to the invention have an extended self-renewal activity and can be expanded without limit.
(2) The hypoxic MSCs according to the invention show low immunogenicity in vivo, and are less susceptible to immune rejection than normoxic MSCs. Thus, the hypoxic MSCs according to the invention and their derivatives are suitable for allogeneic transplantation. The current studies demonstrated that MSCs expanded under hypoxic culture decreased the expression ofNK ligands and inhibited recruitment and cytotoxicity of NK cells, thereby enhancing the ability to survive and engraft into tissues of allogeneic recipients.

Also, the invention provides a cell graft prepared by the aforementioned process.

### Example

### Materials and methods

### MSC isolation, characterization and culture

MSCs were obtained from 2 inbred strains of mice: C57BL/6J (B6) and Balb/c. Male mice 4-6 weeks old were sacrificed by the use of cervical dislocation method. The femurs and tibiae were removed, cleaned of all connective tissue, and placed on ice in 5 mL of α-MEM supplemented with 1 × penicillin/streptomycin. All tibiae and femurs with the ends clipped to expose the marrow were inserted into adapted centrifuge tubes and centrifuged for 1 min at 400g to collect the marrow. The pellet was resuspended in 3 mL of complete medium [CM: α-MEM supplemented with 16.6% fetal bovine serum (FBS), 100 U/mL penicillin, 100 g/mL streptomycin.] with a 21-gauge syringe followed by filtration through a 70-µm nylon mesh filter. The cells from each long bone were plated in 40 mL of CM in a 10-cm dish. After 24 h, nonadherent cells were removed by phosphate-buffered saline (PBS), and 10 mL of fresh CM was added. The adherent cells reached subconfluence after around 2 weeks of incubation (passage 0), and were washed with PBS and detached by incubation in 4 mL of 0.25% trypsin/1 mM ethylenediaminetetraacetic acid (EDTA; Invitrogen, Carlsbad, CA) for 2 min at 37°C. The cells were then expanded by seeding at a density of 50 cells/cm² in CM and subcultured every10 days (passage 1 to 4). The cells used in these studies were at passage 2 to 4. For hypoxic culture, cells were cultured in a gas mixture composed of 94% N2, 5% CO₂, and 1% O₂.For maintenance of the hypoxic gas mixture, an incubator with 2 air sensors, one for CO₂ and the other for O₂, was used; the O₂ concentration was achieved and maintained using delivery of nitrogen gas (N₂) generated from a liquid nitrogen tank or a tank containing pure N₂. If O₂ percentage rose above the desired level, N₂ gas was automatically injected into the system to displace the excess O₂.

### Characterization of MSCs

To analyze cell-surface expression of typical marker proteins, MSCs were harvested by 5 mM EDTA in PBS. Cells were incubated with FITC-conjugated anti-mouse Sca-1, CD11b, CD31, CD34 and CD45, PE-conjugated anti-mouse CD29, CD44, and CD105. Matched isotype antibodies were served as controls (Becton Dickinson, San Jose, CA). Ten thousand labeled cells were acquired and analyzed using a FACScan flow cytometry running CellQuest software (Becton Dickinson). For in vitro differentiation into osteoblasts and adipocytes, cells were seeded at a density of 10⁴/cm² and induced in osteoblast induction medium [OIM, consisted of α-MEM (Gibco, Carlsbad, CA,) supplemented with 10% FBS (Gibco), 10⁻⁸ M dexamethasone (Sigma-Aldrich, St. Louis, MO), 10 mM β-glycerol phosphate (Sigma-Aldrich) and 50 µM ascorbate-2-phosphate (Sigma-Aldrich)] and adipocyte induction medium [AIM, consisted of α-MEM supplemented with 10% FBS, 10⁻⁷ M dexamethasone, 50 µM indomethacin (Sigma-Aldrich), 0.45 mM 3-isobutyl-1-methyl-xanthine (Sigma-Aldrich) and 10 µg/mL insulin (Sigma-Aldrich)] for 3 weeks, respectively. After the appearance of morphologic features of differentiation, cells treated in OIM and AIM were stained for Alizarin Red S (ARS) and Oil-red O, respectively. For quantification of differentiation, the ARS and Oil-red O dyes were extracted for optical measurement at OD 550 nm and 510 nm, respectively. For in vitro differentiation into chondrocytes, 2.5×10⁵ cells were pelleted and continuously cultured in chondrocyte induction medium [CIM, consisted of serum-free α-MEM supplemented with 10⁻⁷ M dexamethasone, 1% (vol/vol) ITS, 50 µM ascorbate-2-phosphate, 1 mM sodium pyruvate, 40 µg /mL (wt/vol) proline and 10 ng/mL (wt/vol) TGF-β1 (R&D systems, Minneapolis, MN)] for 3 weeks. Cells induced in CIM were prepared for Alcian Blue staining.

### Ischemic limb models

The animal research protocol was reviewed and approved by the animal center committee of National Ying-Ming University. Male Balb/c mice at 6 weeks of age were used in the experiments. Under general anesthesia using xylazine and ketamine, a skin incision was made from the knee towards the medial thigh, followed by dissecting away of subcutaneous fat tissue to reveal the underlying femoral artery at the proximal location near the groin. Then the femoral artery was doubly ligated at the midway of the thigh, and 5 mm in length was excised away. Immediately after the resection, 10⁶ culture-expanded MSCs in 100 µL of PBS were injected (i.m.) into the Gracilis muscle of the hind limb.

### Evaluation of limb perfusion

Laser Doppler imaging analysis was performed as described previously (Choi et al., J Biol Chem. 2004;279(47);49430-49438). A laser Doppler perfusion imager (Moor Instruments, Devon, UK) was used for serial noninvasive physiological evaluation of neovascularization. Mice were monitored by serial scanning of surface blood flow of hind limbs on days 0, 3, 7, 14, 21, and 28 after treatment. The digital color-coded images were analyzed to quantify the blood flow in the region from the knee joint to the toe, and mean values of perfusion were calculated.

### Immunohistochemistry and immunofluorescence

For immunohistochemical staining, paraffin-embedded sections were deparaffinized, rehydrated and antigen retrieved by placing sections in Declere working solution (Cell Marque, Austin, TX) in a microwave oven for 20 min. Endogenous peroxidase activity was blocked by 3% hydrogen peroxide. Residual enzymatic activity was removed by washes in PBS, and nonspecific staining was blocked with Ultra V Block for 5 min (Thermo Fisher Scientific, Fremont, CA). Then the sections were reacted with first antibodies (anti-CD31, Santa Cruz Biotechnology, Santa Cruz, CA) overnight at 4°C, washed extensively with PBS, and reacted with corresponding biotinylated secondary antibodies (Vector Laboratories, Burlingame, CA) for 15 min at room temperature and treated with streptavidin-peroxidase (LSAB Kit; Dako, Carpinteria, CA), followed by diaminobenzidine staining. Counterstaining was performed with Mayer's hematoxylin. For immunofluorescence, sections were fixed with 4% paraformaldehyde, reacted with first antibodies, washed extensively with PBS containing 0.1 % Triton X-100, reacted with corresponding DyLight™488 or DyLight™594- conjugated secondary antibodies (Jackson ImmunoResearch Laboratories, West Grove, PA), and counterstained with DAPI. Immunofluorescence was observed with a fluorescence microscope.

### Quantitative RT-PCR

Total RNA from cells was isolated using TRIzol reagent (Invitrogen), and 2 µg of total RNA was reversely transcribed in 20 µL using 3 µg of random primers and 200 U Superscript III RT (Invitrogen). The amplification was carried out in a total volume of 25 µL containing 0.5 µM of each primer, 12.5 µL of LightCycler™-FastStart DNA Master SYBR green I (Roche Indianapolis, IN) and 10 µL of 1:20 diluted cDNA. PCR reactions were performed using a LightCycler 480 Real-time PCR System. The quantification for each mRNA expression was normalized to the housekeeping gene glyceraldehyde-3-phosphate dehydrogenase (GAPDH) using LightCycler 480 Software version 1.5.0 (Roche). Sequences of primer sets were listed in Table 1.

**Table 1. Primer sets for Quantitative PCR**

| **Name** | **Primer sequence** | **Size** | |
|---|---|---|---|
| nectin-2 | F: GGAGGTATTATCGCTGCCATC | 196 | (SEQ ID NO: 1) |
| | R: CCAAGGTGAAGAGTTGAGAGG | | (SEQ ID NO: 2) |
| Pvr | F: GGTGACTTTCCCAACTCTGT | 239 | (SEQ ID NO: 3) |
| | R: GCTGCGATGATGATGACTAC | | (SEQ ID NO: 4) |
| H60c | F: AGATTTCAGTTGCTGCCTCA | 78 | (SEQ ID NO: 5) |
| | R: ACATGTGCAGCAGTGGTTG | | (SEQ ID NO: 6) |
| Rae-1 | F: CCCCAGTATCACCCAGCTTACAT | 168 | (SEQ ID NO: 7) |
| | R: CCCTCCTCTGGCCTCTCCTT | | (SEQ ID NO: 8) |
| Human PVR | F: TGGACGGCAAGAATGTGACC | 116 | (SEQ ID NO: 9) |
| | R: ATCATAGCCAGAGATGGATACC | | (SEQ ID NO: 10) |
| Human ULBP3 | F: AGATGCCTGGGGAAAACAACTG | 151 | (SEQ ID NO: 11) |
| | R: GTATCCATCGGCTTCACACTCACA | | (SEQ ID NO: 12) |
| GAPDH | F: GAAGGTCGGTGTGAACGGA | 241 | (SEQ ID NO: 13) |
| | R: GTTAGTGGGGTCTCGCTCCT | | (SEQ ID NO: 14) |

### NK cytotoxicity assay

For purification of NK cells by MACS, splenocytes were incubated with the NK cell isolation kit (Miltenyi Biotec, Auburn, CA), and cells were sorted by autoMACS (Miltenyi Biotec). NK cell media consisted of RPMI 1640 containing 10% FBS and penicillin/streptomycin. NK cells were expanded in NK cell media in the presence or absence of 1000 U/mL IL-2 for 5 days. The cytotoxic activities of NK cell to MSC were determined using Promega CytoTox 96 Non-Radioactive Cytotoxicity Assay kit to measure lactate dehydrogenase (LDH), a stable cytosolic enzyme that is released upon cell lysis. 5 × 10³ target (T) cells (MSC) were incubated with effector (E) cells (NK) at different E:T ratios (12:1, 6:1, 3:1, 1.5:1) for 4 h; after incubation, supematants were collected and incubated for 30 min with the substrate mix. The reaction was then stopped by adding 1 M acetic acid. Triplicates of each E to T ratio were performed to measure the experimental LDH release. Effector Spontaneous LDH release was measured by incubating the effector cells in the absence of target cells. Target Spontaneous LDH release was measured by incubating the target cells in the absence of effector cells. Target Maximum LDH release was determined by adding lysis solution (0.9 % (v/v) Triton X-100). The amount of LDH release was measured in a plate reader (Bio-Rad Model 3550-UV) and the percentage of cytotoxicity was calculated for each E:T ratio according to the formula:cytotoxicity (%) = 100% ×[ (Experimental - Effector Spontaneous - Target Spontaneous)/ (Target Maximum - Target Spontaneous)].

### Statistical analysis

All values are expressed as mean ± SD. Comparisons between two groups were analyzed by Student *t*-test. A value of p < 0.05 was considered statistically significant.

### Results

### Characterization of mesenchymal stem cells cultured under hypoxic and normoxic conditions

MSCs isolated from both of B6 and Balb/c mice and cultured under hypoxic (1% O₂) and normoxic (air, equal to 20-21% O₂) conditions were characterized using flow cytometric analysis and differentiation assay at passage 3. Both of hypoxic and normoxic MSCs from either B6 or Balb/c expressed markers of MSCs including Sca1, CD29, CD44, CD105, but lacked the expression of markers for haematopoietic cells, such as CD11b, CD34 and CD45, and endothelial markers such as CD31 (Figure 1A and 1B). Moreover, hypoxic and normoxic MSCs from either B6 or Balb/c possessed similar ability to differentiate into osteogenic, adipogenic and chondrogenic lineages (Figure 1C). These data suggest that there are no obvious differences between hypoxic and normoxic MSCs in terms of surface protein profile and differentiation potentials at early passages.

### Allogeneic transplantation of hypoxic MSCs increases limb ischemia improvement compared to normoxic MSCs

The angiogenic potential of MSCs was evaluated in a Balb/c mouse model of hindlimb ischemia. To demonstrate the potential of allogeneic MSCs for ischemic limb therapy, right side femoral artery was ligated and truncated for 5 mm in Balb/c mice, followed by intramuscular injection without cells (control, PBS only), with B6 hypoxic or normoxic MSCs (allogeneic), with Balb/c hypoxic or normoxic MSCs (syngeneic), or with Balb/c hypoxic mouse embryonic fibroblasts (non-MSC). At 1-2 weeks after the injection treatment, the control group showed extensive necrosis of the ischemic hindlimb, which resulted in limb loss by autoamputation. It was shown that at 4 weeks, the control group had seven limb loss (87.5%) and one limb necrosis (12.5%) out of 8 animals (Figure 2A). Similarly, the non-MSC group (MEF Balb/c) had five limb loss (83.3%) and one limb necrosis (16.7%) out of 6 mice (Figure 2A). All of the mice receiving hypoxic MSCs from B6, or hypoxic or normoxic MSCs from Balb/c exhibited limb salvage (70%, 7 of 10 for Balb/c; 70%, 7 of 10 for B6) or limb necrosis (30%, 3 of 10 for Balb/c; 30%, 3 of 10 for B6), while only two mice receiving normoxic MSCs from B6 exhibited limb salvage (20%, 2 of 10), three had limb necrosis (30%, 3 of 10) and five lost their limbs (50%, 5 of 10) (Figure 2A).

Evaluation of blood perfusion by laser Doppler perfusion imaging analysis further revealed that mice receiving hypoxic or normoxic MSCs from either Balb/c or B6 increased in perfusion 1 week after transplantation compared to no cell control or mice receiving non-MSCs(Figure 2B). Mice receiving hypoxic MSCs from B6 or hypoxic or normoxic MSCs from Balb/c increased in perfusion 2 weeks after transplantation compared to those receiving normoxic MSCs from B6 (Figure 2B). Moreover, at 4 weeks after transplantation, the relative ratios of blood flow of the inured limb to the normal limb were 0.926±0.076, 0.972±0.021 and 0.927±0.059 for mice receiving hypoxic MSCs from B6, and hypoxic or normoxic MSCs from Balb/c, respectively, while the ratios were 0.602±0.080, 0.294±0.029, and 0.370±0.012 for mice receiving normoxic MSCs from B6, non-MSC, and no cell transplantation, respectively (Figure 2B). These data suggest ischemic limbs transplanted with hypoxic MSCs from allogeneic donors, or both hypoxic and normoxic MSCs from syngeneic donors recovered with near normal perfusion, while those transplanted with normoxic MSCs from allogeneic donors still have significant limb perfusion impairment.

### Hypoxic MSCs decrease muscle degeneration and fibrosis and increase the angiogenesis of ischemic limb

Histological analysis with hematoxylin and eosin (H&E) staining of the gracilis muscle retrieved at 4 weeks after creation of limb ischemia revealed that mice receiving hypoxic MSCs from B6, or hypoxic or normoxic MSCs from Balb/c maintained normal muscular picture without loss of muscle fibers, while those receiving normoxic MSCs from B6 mice still experienced muscle atrophy with loss of some fibers. However, mice receiving no cell transplantation or non-MSC had marked muscle fiber loss and atrophy with fatty degeneration. Following quantitation of muscle loss by measuring the ratio of non-muscle area to total area in the experimental specimens, we found the ratios of muscle loss were 3.32±1.36%, 3.62±1.01 %, and 3.35±3.04% for mice that received hypoxic MSCs from B6, and hypoxic or normoxic MSCs from Balb/c, respectively, while the ratios were 44.91±1.16%, 53.68±5.95%, and 62.33±2.73% for mice that received normoxic MSCs from B6, non-MSC, and no cell transplantation, respectively (Figure 3A). Similarly, Masson's trichrome staining revealed mice that received hypoxic MSCs from B6 (0.016±0.006%), hypoxic (0.029±0.014%) or nomoxic (0.28±0.14%) MSCs from Balb/c showed no muscle fibrosis, while those that received normoxic MSCs from B6 mice (9.39±1.07%) still experienced some muscle fibrosis (Figure 3B). However, mice that received non-MSC (22.93±3.99%) or no cell transplantation (30.14±11.59%) resulted in marked muscle fibrosis (Figure 3B). Immunohistochemical staining with antibody against CD31 revealed that mice receiving hypoxic MSCs from B6 (173±12/mm²), or hypoxic (189±28/mm²) or normoxic (158±12/mm²) MSCs from Balb/c had significantly enhanced CD31+ capillary density compared with those that received normoxic MSCs from B6 (84±11/mm²), non-MSC (56±10/mm²), or no cell transplantation (60±9/mm²) (Figure 3C). These data demonstrate the therapeutic contribution of allogeneic MSC transplantation to muscle regeneration and that neovascularization was significantly enhanced in MSCs cultured under hypoxic conditions.

### Hypoxic MSCs survive and engraft after transplantation

BrdU tracking system for long-term engraftment revealed a great increase in the presence of hypoxic MSCs from B6 (633±72/mm²), hypoxic (566±71/mm²) or normoxic (620±37/mm²) MSCs from Balb/c, compared to normoxic MSCs from B6 (66±45/mm²) in ischemic tissues at 4 weeks after transplantation (Figure 4A). Double-immunofluorescence for BrdU and CD31 showed some BrdU+ cells were observed in the CD31+ vessels with red blood cells in their lumens (Figure 4B), indicating some transplanted cells were incorporated into neovessels and indeed functioned and contributed to blood perfusion. Double-immuno fluorescence for BrdU and α-smooth muscle actin (α-SMA) or desmin (Figure 4C and 4D) showed some BrdU+ cells were also positive for α-SMA or desmin in the ischemic regions, suggesting some transplanted cells differentiated into muscle tissues. The densities of BrdU+/CD31+ cells were 95±5/mm² for Hyp B6, 95±17/mm² for Hyp Balb/c, 100±9/mm² for Nor Balb/c, and 5±1/mm² for Nor B6; the densities of BrdU+/α-SMA+ were 205±40/mm² for Hyp B6, 215±34/mm² for Hyp Balb/c, 210±35/mm² for Nor Balb/c, and 6±5/mm² for Nor B6, while the densities of BrdU+/desmin+ cells were 180±24/mm² for Hyp B6, 175±11/mm² for Hyp Balb/c, 185±21/mm² for Nor Balb/c, and 10±6/mm² for Nor B6. These results suggest transplanted hypoxic MSCs survived after transplantation, engrafted into mouse tissue, and subsequently induced vascular and muscular networks in the ischemic muscles.

### Hypoxic MSCs decrease NK accumulation in allogeneic recipients

The anti-NKp46 and anti-DX5 antibodies were used to identify NK cells at 28 days and 7 days after transplantation, respectively. While the ischemic muscle tissue injected with allogeneic MSCs cultured under normoxic condition (Nor B6) accumulated NKp46+ and DX5+ NK cells, those injected either with syngeneic MSCs (Nor Balb/c and Hyp Balb/c) or allogeneic MSCs cultured under hypoxic condition (Hyp B6) significantly reduced NKp46+ or DX5+ NK accumulation (Figure 5A). However, immunofluorescence with antibodies against CD8, CD4, CD 14, CD86, or TCRαβ showed that both hypoxic and normoxic MSCs from either B6 or Balb/c did not accumulate CD8+ or CD4+ T cells, CD14+ macrophages, CD86+ dendritic cells, or NKT cells (Figure 5B), suggesting the involvement of NK accumulation in the killing of normoxic MSCs in allogeneic recipients. To demonstrate this, we evaluated the effects of transplanted normoxic MSCs on ameliorating limb ischemia in allogeneic recipients without and with NK depletion. NK depletion via co-injection with neutralizing antibodies against NK1.1 or asialo GM1 greatly improved normoxic MSCs-mediated improvement of limb ischemia in allogeneic recipients (Figure 5C). These data suggest the involvement ofNK-mediated lysis in the rejection of allogeneic MSCs after transplantation.

### Hypoxic MSCs decrease expression of ligands for NK activation and NK lysis

Quantitative RT-PCR analyses revealed that hypoxic MSCs reduced the expression of NK ligands, such as H60c, nectin-2, Pvr and Rae1 (Figures 5D), which are important for NK accumulation and activation, or known for NK against hematopoietic progenitor cells, despite that the Rae1 levels were low both in hypoxic and normoxic MSCs. Moreover, decreased expression of these ligands in MSCs from long-term hypoxic culture was also observed in MSCs from 48 h of hypoxic culture (Figure 5E). Hypoxic culture not only suppressed the expression of these ligands, but also suppressed NK-mediated lysis. As shown in Figure 5F, NK in the presence of IL-2 induced 84.15% lysis of normoxic MSCs, while only inducing 0.44% lysis of hypoxic MSCs. Similar results were obtained with experiments using MSCs from Balb/c and NK cells from B6 (data not shown).

### Pretreatment of normoxic MSCs with anti-NK ligand antibodies abrogates NK lysis and increases the ability to improve limb ischemia

Our data strongly supported the involvement of NK ligands in NK lysis of allogeneic normoxic MSCs. It would be interesting to know whether blocking of NK ligands in MSCs or depleting host receptors for NK ligands would abrogate NK-mediated lysis of allogeneic normoxic MSCs, and thereby promote the ability of allogeneic normoxic MSCs in enhancing angiogenesis and preventing limb amputation in ischemia limbs. We therefore pretreated normoxic B6 MSCs with neutralizing of antibodies against H60c, nectin-2, Pvr or Rae1 for 30 min or pretreated NK with DNAM1 or NKG2D blocking antibodies, and analyzed the responses of MSCs to NK-mediated lysis. Normoxic MSCs pre-treated with neutralizing antibodies against H60c, nectin-2, or Pvr decreased NK lysis compared to those pre-treated with isotype IgG or antibodies against Rae1 (Figure 6A). Consistently, NK cells pre-treated with anti-DNAM1 or anti-NKG2D antibodies also decreased lysis ability (Figure 6A). Normoxic MSCs pre-treated with neutralizing antibodies against H60c, nectin-2, or Pvr or co-injected with neutralizing antibodies against DNAM1 or NKG2D, increased the ability to enhance blood perfusion (Figure 6B) and restore limb structures (Figure 6C), compared to those pre-treated with isotype IgG. In contrast, hypoxic MSCs pretreated or co-injected with these blocking antibodies did not further increase the ability to enhance blood perfusion (Figure 6B). Interestingly, we found the NK accumulation induced by allogeneic transplantation with normoxic MSCs was inhibited by pretreatment with neutralization antibodies against H60c, nectin-2 orPvr or co-injection with antibodies against DNAM1 or NKG2D, compared to pretreatment with isotype IgG (Figure 6D). Moreover, we found the engraftment of normoxic MSCs to CD31⁺, α-SMA⁺ and Desmin⁺ cells in allogeneic recipients was enhanced by pretreatment with neutralizing antibodies against H60c, nectin-2 or Pvr or co-injection with antibodies against DNAM1 or NKG2D, compared to pretreatment with isotype IgG (Figure 6E and 6F). These data together suggest the strong involvement ofNK ligands in NK-mediated lysis of MSCs, and failure of blood perfusion enhancement after allogeneic transplantation of normoxic MSCs.

To apply the current mouse findings to future human clinical use, we compared the expression of human NK ligands in human MSCs without and with exposure to hypoxic conditions. We found that human MSCs cultured under normoxic conditions expressed significant mRNA levels ofNK ligands such as PVR (human DNAM-1 ligand) and ULBP3 (human NKG2D ligand), while short-term exposure of human MSCs to hypoxia (1% O₂) inhibited the expression of these ligands (Figures 7A). Consistently, allogeneic NK cells-mediated lysis was observed in human normoxic MSCs but not in human hypoxic MSCs, which was blocked by pretreatment of MSCs with antibodies against PVR or ULBP3 or NK cells with antibodies against DNAM1 andNKG2D (Figures 7B and 7C). The above data suggest that the findings observed in the mouse model may be applied for future human clinical use.

### Short-term exposure to hypoxic conditions reduced expression of NK ligands in human and mouse MSCs

Human and B6 mouse MSCs cells were used in these studies. Human and mouse MSCs were expanded under normoxic conditions (21% O₂) for several weeks. These cells were reseeded at a density of 5 × 10³ to 10⁴ cells/cm² and moved to hypoxic conditions with O₂ concentrations of 0%, 1%, 2.5%, and 7% (as compared to a low density of 10 to 4000 MSCs/cm² used in US20110129918A1). For maintenance of the hypoxic gas mixture, an incubator with 2 air sensors, one for CO₂ and the other for O₂, was used; the O₂ concentration was achieved and maintained using delivery of nitrogen gas (N₂) generated from a liquid nitrogen tank or a tank containing pure N₂. If O₂ percentage rose above the desired level, N₂ gas was automatically injected into the system to displace the excess O₂.The cells were retrieved 2 days after incubation. Total RNA from cells was isolated using TRIzol reagent (Invitrogen), and 2 µg of total RNA was reversely transcribed in 20 µL using 3 µg of random primers and 200 U Superscript III RT (Invitrogen). The amplification was carried out in a total volume of 25 µL containing 0.5 µM of each primer, 12.5 µL of LightCycler™-FastStart DNA Master SYBR green I (Roche Indianapolis, IN) and 10 µL of 1:20 diluted cDNA. PCR reactions were performed using a LightCycler 480 Real-time PCR System. The quantification for each mRNA expression was normalized to the housekeeping gene glyceraldehyde-3-phosphate dehydrogenase (GAPDH) using LightCycler 480 Software version 1.5.0 (Roche).

Mouse MSCs expanded under normoxic conditions expressed high levels of NK DNAM-1 ligands, Pvr and nectin-2, and NKG2D ligand, H60c. In addition, it was also shown that short-term exposure to hypoxic conditions for 2 days with O₂ concentrations around 0 to 7% significantly reduced the expression of H60c, nectin-2 and Pvr (Figures 8A, 8B and 8C). Similarly, human MSCs expanded under normoxic conditions expressed high levels of NK DNAM-1 ligand, PVR, and NKG2D ligand, ULBP3, whereas short-term exposure to hypoxic conditions with O₂ concentrations around 0% to 7% for 2 days significantly reduced the expression of PVR and ULBP3 (Figures 8D and 8E). These results suggest short-term exposure of MSCs to hypoxia reduces the expression ofNK ligands.

In summary, allogeneic hypoxic MSC therapy according to the invention has been tested in an immune-competent animal model as a preclinical study to prove its therapeutic potential and immune benefit, such as in the treatment of severe ischemic diseases. The results show that MSCs cultured under hypoxic conditions can be used for allogeneic transplantation.

## Claims

1. A process for reducing the expression of a NK ligand of a cell graft made from mesenchymal stem cells (MSCs), comprising culturing said MSCs under low oxygen conditions ranging from 0% to about 7 % oxygen wherein the NK ligand is selected from the group consisting of H60c, nectin-2, PVR, ULBP3 and combinations thereof.

2. The process of claim 1, wherein the cell graft induces reduced or no graft rejection when transplanted onto a subject.

3. The process of claim 1, wherein the low oxygen condition ranges from 0% to about 2.5% oxygen.

4. The process of claim 3, wherein the low oxygen condition is about 1% oxygen.

5. The process of claim 1, wherein said cell graft is allogeneic with the subject.

6. The process of claim 1, wherein said MSCs preserve early stem cell properties, maintain normal karyotyping, and will not form tumor after transplantation.


SEQUENCE LISTING

<110>  Taipei Veterans General Hospital

<120>  PREPARATION OF CELL TRANSPLANT

<130>  IV0188/HSC0001EP

<150>  61/583,367
<151>  2012-01-05

<160>  14

<170>  PatentIn version 3.5

<210>  1
<211>  21
<212>  DNA
<213>  Artificial Sequence

<220>
<223>  nectin-2 F

<400>  1
ggaggtatta tcgctgccat c                                                21

<210>  2
<211>  21
<212>  DNA
<213>  Artificial Sequence

<220>
<223>  nectin-2 R

<400>  2
ccaaggtgaa gagttgagag g                                                21

<210>  3
<211>  20
<212>  DNA
<213>  Artificial Sequence

<220>
<223>  Pvr F

<400>  3
ggtgactttc ccaactctgt                                                  20

<210>  4
<211>  20
<212>  DNA
<213>  Artificial Sequence

<220>
<223>  Pvr R

<400>  4
gctgcgatga tgatgactac                                                  20

<210>  5
<211>  20

<212>  DNA
<213>  Artificial Sequence

<220>
<223>  H60c F

<400>  5
agatttcagt tgctgcctca                                                    20


<210>  6
<211>  19
<212>  DNA
<213>  Artificial Sequence

<220>
<223>  H60c R

<400>  6
acatgtgcag cagtggttg                                                     19


<210>  7
<211>  23
<212>  DNA
<213>  Artificial Sequence

<220>
<223>  Rae-1 F

<400>  7
ccccagtatc acccagctta cat                                                23


<210>  8
<211>  20
<212>  DNA
<213>  Artificial Sequence

<220>
<223>  Rae-1 R

<400>  8
ccctcctctg gcctctcctt                                                    20


<210>  9
<211>  20
<212>  DNA
<213>  Artificial Sequence

<220>
<223>  Human PVR F

<400>  9
tggacggcaa gaatgtgacc                                                    20


<210>  10
<211>  22
<212>  DNA
<213>  Artificial Sequence

<220>
<223>  Human PVR R

<400>  10
atcatagcca gagatggata cc                                              22


<210>  11
<211>  22
<212>  DNA
<213>  Artificial Sequence

<220>
<223>  Human ULBP3 F

<400>  11
agatgcctgg ggaaaacaac tg                                              22


<210>  12
<211>  24
<212>  DNA
<213>  Artificial Sequence

<220>
<223>  Human ULBP3 R

<400>  12
gtatccatcg gcttcacact caca                                            24


<210>  13
<211>  19
<212>  DNA
<213>  Artificial Sequence

<220>
<223>  GAPDH F

<400>  13
gaaggtcggt gtgaacgga                                                  19


<210>  14
<211>  20
<212>  DNA
<213>  Artificial Sequence

<220>
<223>  GAPDH R

<400>  14
gttagtgggg tctcgctcct                                                 20